# FASCICULE DE BREVET EUROPEEN

(11) **EP 1 194 450 B1**
(45) Date de publication et mention de la délivrance du brevet: **20.10.2004**
(21) Numéro de dépôt: 00953242.5
(22) Date de dépôt: 13.07.2000
(51) Int. Cl.: C07K 16/10, A61K 39/12, A61P 31/14, C12N 5/12

(54) **VACCIN INACTIVE CONTRE LA CALICIVIROSE FELINE**
INAKTIVIERTER IMPFSTOFF GEGEN KATZEN CALICIVIRUS
INACTIVATED VACCINE AGAINST FELINE CALICIVIRUS DISEASE

(30) Priorité: 16.07.1999 FR 9909420; 11.02.2000 FR 0001759
(43) Date de publication de la demande: 10.04.2002
(73) Titulaire: MERIAL, 69002 Lyon (FR)
(72) Inventeur: POULET, Hervé, F-69003 Lyon (FR); GOUTEBROZE, Sylvain, Gabriel, F-69007 Lyon (FR)
(74) Mandataire: Nargolwalla, Cyra
(86) Numéro de dépôt international: PCT/FR2000/002050
(87) Numéro de publication internationale: WO 2001/005835

(56) Documents cités:
- WO-A-98/56929
- DATABASE MEDLINE [en ligne] US NATIONAL LIBRARY OF MEDICINE (NLM), BETHESDA, MD, US; POULET H ET AL: "Comparison between acute oral/respiratory and chronic stomatitis/gingivitis isolates of feline calicivirus: pathogenicity, amtigenic profile and cross-neutralization studies." XP002138104 & ARCHIVES OF VIROLOGY (2000) 145 (2) 243-61,

## Description

La présente invention concerne l'utilisation de souches particulières de calicivirus félins pour la réalisation de préparations immunogènes et de vaccins, notamment inactivés ou de sous-unités, contre la calicivirose féline. Ces préparations immunogènes et ces vaccins peuvent également être combinés avec des préparations immunogènes ou des vaccins préparés sur la base d'autres agents pathogènes félins, pour la réalisation de préparations immunogènes et de vaccins multivalents.

Les calicivirus félins (en anglais Feline Calicivirus ou FCV) ont été décrits pour la première fois en 1957 (Fastier L.B. Am. J. Vet. Res. 1957. **18.** 382-389). Les calicivirus félins sont avec les virus herpès félins les deux principales sources des affections virales du tractus respiratoire supérieur chez le chat. Les virus FCV touchent un grand nombre d'animaux, avec des taux de portage du FCV de l'ordre de 15 à 25 %, et une séroprévalence anti-FCV de 70 à 100 % (Coutts *et al*. Vet. Rec. 1994. **135.** 555-556 ; Ellis T.M. Australian Vet. J. 1981. **57**. 115-118; Harbour *et al*. Vet. Rec. 1991. **128.** 77-80 ; Reubel *et al*. Feline Dendistry 1992. **22.** 1347-1360). Après une phase initiale d'hyperthermie, ces affections respiratoires s'accompagnent généralement d'ulcérations buccales (palais, langue, lèvres, nez), de rhinite, de conjonctivite, éventuellement d'anorexie et d'asthénie. Les virus FCV peuvent également causer des pneumonies, des entérites, et des douleurs articulaires (syndrome de boiterie).

La transmission du virus FCV ne se fait qu'horizontalement, il n'y a pas de transmission verticale de la mère à son chaton lors de la gestation (Johnson R.P. Res. Vet. Sci. 1984. **31**. 114-119). La transmission du FCV se fait par contact entre animaux infectés et animaux sains ou par voie aérienne lors d'éternuements (Wardley RC. Arch. Virol. 1976. **52.** 243-249).

Les calicivirus félins sont des virus nus de la famille des *Caliciviridae*, ils sont pourvus d'un ARN positif simple brin, d'une taille d'environ 7,7 kilo paires de bases (kpb) (Radford *et al*. Proc. 1^{st} Int. Symp. Caliciviruses ESVV 1997. 93-99).

Comme de nombreux virus à ARN, une grande hétérogénéité existe au sein de la population virale de FCV. Les variations antigéniques, mises en évidence dès le début des années 70 par des expériences de séroneutralisations croisées, permettent de classer les FCV en plusieurs souches virales ou quasi-espèces (Radford *et al*. Proc. 1^{st} Int. Symp. Caliciviruses ESVV 1997. 93-99).

Plusieurs souches de FCV ont été identifiées et isolées, notamment la souche F9 (déposée auprès de l'American Type Culture Collection ou ATCC sous le numéro d'accès VR-782), la souche 2280 (ATCC VR-2057), la souche KCD (ATCC VR-651), et la souche CFI (ATCC VR-654).

La vaccination contre le FCV a été mise en place depuis la fin des années 70 à partir de souches FCV atténuées, principalement la souche F9 isolée aux USA en 1958 par Bittle (Bittle *et al*. Am. J. Vet. Res. 1960. **21**. 547-550) ou des souches dérivées de F9 par passage *in vitro* ou *in vivo* (" F9-like ").

Des vaccins inactivés sont aussi disponibles. Ils utilisent principalement la souche 255, isolée aux USA en 1970 chez un chat présentant une pneumonie (Kahn et Gillepsie. Cornell Vet. 1970. **60.** 669-683 ; Povey *et al.* J. Am. Vet. Med. Assoc. 1980. **177.** 347-350), la souche 2280 isolée aux USA en 1983 chez un chat atteint de boiterie (Pedersen *et al*. Fel. Prac. 1983. **13.** 26-35 ; Pedersen N.C. et Hawkins K.F. Vet. Microbiol. 1995. **47.** 141-156).

Du fait de la dérive antigénique dans le temps, les antisérums produits contre des souches vaccinales isolées dans les années 60-70, telles que les souches F9, 255, ou 2280, ne neutralisent que peu d'isolats des années 90. Par exemple, le sérum anti-F9 neutralise 43 % des isolats américains de la période 1990-96, contre 56 % pour la période 1980-89 et 86 % pour la période 1958-79, et seulement 10 % des isolats anglais de la période 1990-96 (Lauritzen *et al*. Vet. Microbiol. 1997. **56.** 55-63). C'est pourquoi les vaccins atténués et inactivés élaborés à partir d'anciennes souches de FCV n'offrent plus à présent une protection suffisante face aux souches FCV récentes.

La présente invention a pour objectif la mise en évidence de nouvelles souches FCV induisant chez le chat des anticorps ayant un large spectre de neutralisation croisée.

Un autre objectif de l'invention est l'élaboration de préparations immunogènes et de vaccins contre la calicivirose féline à partir de ces souches FCV.

Un autre objectif encore de l'invention est l'élaboration de préparations immunogènes multivalentes et de vaccins multivalents contre la calicivirose féline et contre au moins un autre pathogène félin.

La déposante a sélectionné quatre souches FCV obtenues par écouvillonnages pharyngés pratiqués en France, au Royaume-Uni et aux USA sur des chats présentant des signes d'infection par calicivirus félins. Il s'agit respectivement de la souche G1 (déposée à la Collection Nationale de Cultures de Microorganismes (ou CNCM) de l'Institut Pasteur, Paris, France, sous le numéro d'accès I-2167) et de la souche 431 (déposée à la CNCM sous le numéro d'accès I-2166), toutes les deux déposées le 12 mars 1999. Les deux dernières souches sont américaines et désignées RMI6 et RMI9. La souche FCV G1 isolée en France ne correspond pas à la souche FCV isolée au Royaume-Uni en 1978 par Tohya Y: (Tohya Y. *et al*. Jpn. J. Sci., 1990, **52**, 955-961) et nommée également G1.

La sélection des souches FCV 431, G1, RMI6 et RMI9 a été réalisée par des tests de séroneutralisation croisée vis-à-vis des isolats FCV d'un panel de référence. Ce panel de référence est composé de 18 isolats actuels de FCV prélevés sur des chats présentant des signes d'infection par calicivirus félin et provenant de trois zones géographiques distinctes. 7 isolats sont américains, ces isolats sont identifiés RMI1, RMI2, RMI3, RMI5, RMI6, RMI7 et RMI9. 7 isolats sont français, ils sont désignés A2, F1, G1, G3, F3031, H3-2 et H1-4. Les 4 derniers isolats sont anglais, ils sont désignés 431, 388b, 337 et J5.

Les souches du panel et les souches RMI6 et RMI9 sont accessibles auprès de la déposante sur simple demande.

Elles ont aussi été publiées dans un article de la revue « Archives of Virology » (Poulet *et al*. Arch. Virol. février 2000.**145(2)**. 243-261), disponible en ligne sur Intemet à la date de dépôt auprès de l'éditeur.

Lors de tests de séroneutralisations croisées entre les 18 isolats de FCV du panel de référence, il s'est avéré de manière surprenante que l'anti-sérum de l'isolat 431 neutralise 14 des 17 isolats hétérologues du panel de référence (le titre homologue de séroneutralisation n'est pas pris en compte). Par comparaison les anti-sérums des souches vaccinales " historiques " 255 et F9 ne neutralisent chacun que 2 des 18 isolats du panel.

De manière inattendue, la déposante a donc trouvé avec la souche FCV 431 une souche dominante qui peut être utilisée pour la protection des félidés et en particulier des chats contre la plupart des souches FCV. Grâce au panel de souches FCV divulguées ici, il est possible à l'homme du métier de sélectionner d'autres souches FCV dominantes. Par équivalence l'invention couvre aussi à travers la souche FCV 431 les souches FCV équivalentes à cette dernière, ayant des anticorps à large spectre de neutralisation croisée.

Il y a équivalence lorsque l'anti-sérum d'une souche FCV séroneutralise au moins 13 des 18 isolats hétérologues du panel de référence (c'est-à-dire incluant FCV 431), préférentiellement lorsqu'il séroneutralise au moins 14 des 18 isolats hétérologues du panel de référence, de manière encore plus préférée lorsqu'il séroneutralise au moins 15 des 18 isolats hétérologues du panel de référence.

On considère généralement qu'une souche de FCV séroneutralise une autre souche FCV lorsque le titre hétérologue de séroneutralisation est supérieur ou égal à 1,2 log₁₀ VN₅₀ (Povey C. et Ingersoll J., Infection and Immunity, 1975, **11**, 877-885). La déposante a pris cette valeur comme seuil de positivité. Cependant, les résultats de séroneutralisation croisée obtenus par un isolat FCV ayant un titre homologue de séroneutralisation inférieur ou égal à 2 log₁₀ VN₅₀ ne peuvent être interprétés.

Une seconde méthode pour établir l'équivalence d'une souche FCV vis-à-vis de la souche FCV 431 est d'utiliser des anticorps monoclonaux spécifiques de la souche FCV 431 et de tester par immunofluorescence indirecte (IFI) la souche FCV candidate. La déposante a ainsi réussi à produire plusieurs anticorps monoclonaux qui se sont avérés spécifiques de la souche 431. L'un d'eux a été nommé 44. Il y a équivalence si il y a une réactivité en immunofluorescence avec des anticorps monoclonaux spécifiques de 431, par exemple avec l'anticorps monoclonal 44. Cet anticorps monoclonal et l'hybridome correspondant sont disponibles auprès de la déposante sur simple demande et sont aussi divulgués dans l'article de Poulet *et al*. Arch. Virol. 2000. **145**. 1-19. L'hybridome correspondant a également été déposé le 11 août 1999 à la CNCM sous le numéro d'accès I-2282. Il va de soit cependant que l'homme du métier est parfaitement à même de produire des anticorps monoclonaux par les techniques classiques et de sélectionner, par rapport au panel, ceux qui sont spécifiques de la souche 431.

La présente invention a donc pour premier objet des préparations immunogènes et des vaccins comprenant un calicivirus félin (FCV) ayant la particularité d'être reconnu par l'anticorps monoclonal 44, par exemple calicivirus félin souche 431, ou la protéine de capside d'un tel FCV dans un véhicule ou excipient acceptable sur le plan vétérinaire, et de préférence en présence d'un adjuvant. La notion de préparation immunogène recouvre ici toute préparation susceptible, une -fois administrée au chat, d'induire une réponse immunitaire dirigée contre le pathogène félin considéré. Par vaccin, on entend une préparation capable d'induire une protection efficace.

Les autres souches FCV G1, RMI6 et RMI9 ont été choisies pour leur complémentarité vis-à-vis de la souche FCV 431, à savoir que la combinaison des anti-sérums de 431 et d'un de ces trois FCV séroneutralisent 100% des isolats du panel de référence, c'est-à-dire que ces trois souches FCV ont un titre homologue de séroneutralisation supérieur ou égal à 2 log₁₀ VN₅₀ et des titres de séroneutralisation hétérologues supérieurs ou égaux à 1,2 log₁₀ VN₅₀ vis-à-vis des isolats FCV du panel de référence contre lesquels l'anti-sérum 431 ne séroneutralise pas ou faiblement (valeur inférieure à 1,2 log₁₀ VN₅₀). L'invention couvre également les souches FCV équivalentes ayant la même complémentarité vis-à-vis de la souche FCV 431. On peut aussi produire et sélectionner des anticorps monoclonaux spécifiques de ces souches, en particulier de G1, ce qui permet ensuite de déterminer des équivalents sur cette autre base.

L'invention a donc pour deuxième objet des préparations immunogènes et des vaccins comprenant, outre des antigènes de la souche FCV 431 ou l'un de ces équivalents selon l'invention, des antigènes d'au moins une autre souche FCV, notamment une souche complémentaire, en particulier choisie parmi le groupe comprenant G1, RMI6, RMI9, ce qui inclut leurs équivalents, dans un véhicule ou excipient acceptable sur le plan vétérinaire, et éventuellement un adjuvant. De préférence, les antigènes provenant de la ou des autres souches FCV comprennent du virus inactivé ou des sous-unités.

L'invention a en particulier pour objet l'association des deux souches FCV 431 et G1 pour la réalisation de préparations immunogènes ou de vaccins inactivés ou de sous-unités.

De façon surprenante, la combinaison des deux souches FCV G1 et 431 provoque avantageusement un effet synergique. Lors d'études sur la complémentarité des souches FCV G1 et 431, les réponses immunitaires induites par G1 seule, 431 seule ou l'association des deux (G1 + 431) ont été comparées. Le groupe d'animaux ayant été immunisés par la combinaison des deux souches FCV G1 et 431 a bénéficié d'une meilleure protection clinique.

La culture et la propagation des virus FCV se fait de préférence sur des cellules félines, plus particulièrement sur cellules rénales félines de Crandell-Reese (en anglais Crandell-Reese Feline Kidney ou CRFK accessible auprès de l'American Type Culture Collection sous le numéro CCL-94) avec une multiplicité d'infection (moi) de 2 à 0,01 doses infectantes 50 % en culture de cellules (DICC₅₀) par cellule, de préférence 0,5 DICC₅₀/cellule.

Après récolte et clarification, les virus FCV destinés à produire une préparation immunogène inactivée ou un vaccin inactivé sont inactivés par un traitement chimique (e.g. formol ou formaldéhyde, β-propiolactone, éthylèneimine, éthylèneimine binaire (BEI)) et/ou thermique. De préférence, les virus selon l'invention sont inactivés par action de l'éthylèneimine formée extemporanément à partir du bromoéthylamine (BEA). Les particules virales peuvent être concentrées par des techniques classiques de concentration, notamment par ultrafiltration et, éventuellement ensuite purifiées par des moyens de purification classiques, notamment les techniques de gel-filtration ou de précipitations sélectives en particulier en présence de polyéthylène glycol (PEG). Une purification peut aussi être réalisée, sans concentration préalable.

Pour l'élaboration d'une préparation immunogène ou d'un vaccin inactivé ou de sous-unités, les particules virales sont reprises dans un véhicule ou excipient acceptable sur le plan vétérinaire, et éventuellement additionnées d'un adjuvant. La quantité d'antigène équivaut notamment à un titre avant inactivation d'environ 10⁵ à environ 10¹⁰ DICC₅₀ par dose, de préférence d'environ 10⁸ à environ 10⁹ DICC₅₀ par dose.

Pour adjuver les préparations immunogènes et vaccins selon l'invention, on peut utiliser à titre d'adjuvant (1) de l'hydroxyde d'alumine, (2) un polymère de l'acide acrylique ou méthacrylique, un polymère d'anhydride maléique et de dérivé alcényle, ou (3) formuler la préparation immunogène ou vaccin sous forme d'une émulsion huile-dans-l'eau en particulier l'émulsion SPT décrite p. 147 « Vaccine Design, The Subunit and Adjuvant Approach » edited by M. Powell, M. Newman, Plenum Press 1995, et l'émulsion MF59 décrite p183 du même ouvrage.

L'émulsion huile-dans-l'eau peut notamment être à base d'huile de paraffine liquide légère (type Pharmacopée européenne) ; d'huile isoprénoide telle que le squalane, le squalène ; d'huile résultant de l'oligomérisation d'alcènes, en particulier d'isobutène ou de decène ; des esters d'acides ou d'alcools à groupement alkyle linéaire, plus particulièrement les huiles végétales, l'oléate d'éthyle, le di(caprylate / caprate) de propylène glycol, le tri(caprylate / caprate) de glycérol, le dioléate de propylène glycol ; des esters d'acides ou d'alcools gras ramifiés, en particulier des esters de l'acide isostéarique. L'huile est utilisée en association avec des émulsifiants pour former l'émulsion. Les émulsifiants sont de préférence des tensio-actifs non ioniques en particulier les esters de sorbitan, de mannide, de glycérol, de polyglycérol, de propylène glycol et de l'acide oléique, isostéarique, ricinoléique, hydroxystéarique, éventuellement éthoxylés, les blocs copolymères polyoxypropylène-polyoxyéthylène en particulier les Pluronic®, notamment L121.

Les polymères de l'acide acrylique ou méthacrylique sont réticulés notamment par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2 909 462 décrivant de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. Vinyles, allyles et autres groupes éthyléniquement insaturés. Les radicaux insaturés peuvent eux-mêmes contenir d'autres substituants, tel que méthyl. Les produits vendus sous la dénomination Carbopol® (BF Goodrich, Ohio, USA) sont particulièrement appropriés. Ils sont réticulés par un allyl saccharose ou par de l'allylpentaérythritol. Parmi eux, on peut citer les Carbopol® 974P, 934P et 971P.

Parmi les copolymères d'anhydride maléique et de dérivé alcényle, on préfère les EMA® (Monsanto) qui sont des copolymères d'anhydride maléique et d'éthylène, linéaires ou réticulés, par exemple réticulés par du divinyléther. On peut se référer à J. Fields *et al*., Nature, **186**: 778-780, 4 juin 1960. Sur le plan de leur structure, les polymères d'acide acrylique ou méthacrylique et les EMA® sont formés de préférence de motifs de base de formule suivante : 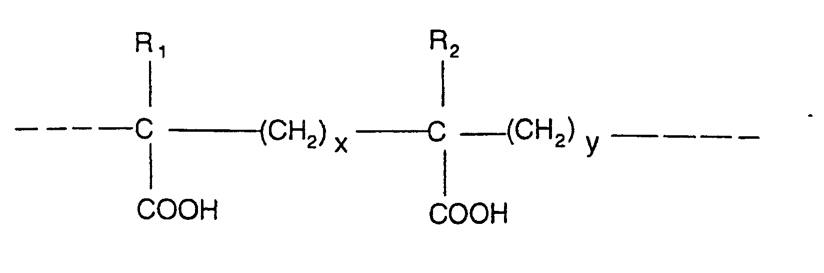 dans laquelle :
- R₁ et R₂, identiques ou différents, représentent H ou CH₃
- x = 0 ou 1, de préférence x = 1
- y = 1 ou 2, avec x + y = 2

Pour les EMA®, x = 0 et y = 2. Pour les carbomères, x = y = 1.

La concentration en polymère dans la composition vaccinale finale sera de 0,01 % à 1,5 % P/V, plus particulièrement de 0,05 à 1 % P/V, de préférence de 0,1 à 0,4 % P/V.

On peut bien entendu aussi combiner du virus inactivé et des sous-unités d'une même souche de FCV conforme à l'invention et/ou de différentes souches de FCV conformes à l'invention.

L'invention a aussi pour objet un vaccin multivalent comprenant une valence calicivirus féline inactivée, comprenant au moins la souche FCV 431, ce qui inclut ces équivalents, et éventuellement au moins une autre souche FCV, notamment une souche complémentaire au sens de l'invention, en particulier choisie parmi G1, RMI6 et RMI9, et au moins une valence pour un autre pathogène félin, dans un véhicule ou excipient acceptable sur le plan vétérinaire et de préférence avec un adjuvant, notamment l'un de ceux décrits précédemment. On peut de la même manière réaliser des vaccins multivalents à base de sous-unités.

Lesdits pathogènes félins sont notamment choisis parmi le groupe comprenant le virus de la rhinotrachéite féline ou virus herpès félin (FHV), le virus de la leucémie féline (Feline Leukemia Virus ou FeLV), le virus de la panleucopénie féline ou parvovirus félin (FPV), le virus de la péritonite infectieuse féline (FIPV), le virus de l'immunodéficience féline (FIV), le virus de la rage, *Chlamydia.*

Les vaccins FCV selon l'invention peuvent être mélangés extemporanément avec la ou les autres valences félines qui peuvent être sous forme de vaccins vivants atténués, inactivés, sous-unitaires, recombinés ou polynucléotidiques.

L'invention a donc également pour objet un kit ou ensemble de vaccination multivalent comprenant, conditionnées séparément, une valence FCV selon l'invention dans un véhicule ou excipient acceptable sur le plan vétérinaire, et de préférence avec un adjuvant, et au moins une valence d'un autre pathogène félin. La valence FCV peut servir de solvant pour une autre valence féline, notamment valence atténuée, recombinée ou polynucléotidique se présentant sous forme lyophilisée.

Conformément à une modalité de l'invention, on peut produire des préparations immunogènes ou des vaccins de sous-unité, par extraction de la capside à partir du virus, avec éventuellement inactivation avant ou après l'extraction. Ces préparations et vaccins comprennent donc comme principe actif unique ou non, un tel produit d'extraction contenant majoritairement de la protéine capside et éventuellement des sous-fragments, éventuellement inactivé, produit à partir des souches selon l'invention, en particulier la souche 431, ce qui inclut ses équivalents, éventuellement aussi à partir d'une autre souche FCV, notamment G1 ou équivalents. Ces préparations et vaccins sous-unitaires sont avantageusement adjuvés, par exemple comme décrit supra. On peut aussi mélanger préparation ou vaccin inactivé entier et préparation ou vaccin sous-unitaire.

L'invention a aussi pour objet une préparation immunogène ou un vaccin basé sur la souche G1, notamment inactivé ou sous-unitaire d'extraction.

L'invention a également pour objet une méthode d'immunisation des chats, contre les maladies dues aux calicivirus félins.

Cette méthode d'immunisation comprend l'administration d'un vaccin FCV inactivé, sous-unitaire ou multivalent associé selon l'invention à des chats. L'administration dudit vaccin peut se faire notamment par la voie parentérale, de préférence par la voie sous-cutanée ou intramusculaire.

L'homme de l'art possède les compétences nécessaires pour définir précisément le nombre d'injection et les doses de chaque vaccin à utiliser pour chaque protocole de vaccination.

Les volumes de dose peuvent être notamment compris entre 0,2 et 2 ml, de préférence de l'ordre de 1 ml.

L'invention va être maintenant décrite plus en détail à i'aide de modes de réalisation pris à titre d'exemples non limitatifs, donnant un tableau des titres de séroneutralisation croisée.

### Exemples :

### Exemple 1 : Isolats viraux et hybridomes

Les virus calicivirus félins (FCV) ont été obtenus par écouvillonnages pharyngés pratiqués sur des chats présentant des signes d'infection par calicivirus félins.
Ces FCV ont différentes origines géographiques.
Les souches FCV 431, 337, J5, 388b, 220 et 393 ont été isolées au Royaume-Uni et fournies par le Professeur O. Jarrett de l'Université de Glasgow, UK.
Les souches FCV A2, G1, G3, F3031, F1, H3-2 et H1-4 ont été isolées en France par la déposante.
Les souches FCV RMI1, RMI2, RMI3, RMI5, RMI6, RMI7 et RMI9 ont été isolées aux USA par la déposante.
Les prélèvements pharyngés sont collectés dans 2 ml de milieu minimum Eagle modifié par Dulbecco (DMEM, Gibco BRL), supplémenté de 5 % de sérum de veau foetal (Bayer Diagnostic), d'antibiotiques, plus particulièrement de 50 mg/l de gentamycine. Chaque isolat est congelé à -70°C en attendant d'être testé.
L'anticorps monoclonal 44, provenant de l'hybridome identifié 431 2 0 17 E9 T, est spécifique de la souche FCV 431.

### Exemple 2 : Amplification des isolats viraux

Des cellules de lignée de rein de chat (Crandell-Reese Feline Kidney ou CRFK N° ATCC CCL-94, Crandell *et al.* ln Vitro 1973. **9.** 176-185) sont mises en culture en plaque de 96 puits ou en Falcon 25 cm² (Falcon) avec du milieu DMEM supplémenté de 5 % de sérum de veau foetal, contenant environ 100 000 cellules par ml. Les cellules sont cultivées à +37°C en atmosphère contenant 5% de CO₂. Après 3 jours la couche cellulaire arrive à confluence. Le milieu de culture est alors remplacé par du milieu DMEM sans sérum mais additionné de 50 mg/l de gentamycine et les aliquotes décongelées des isolats viraux FCV (exemple 1) sont ajoutées à raison d'un volume de 100 µl de dilutions sériées de quart en quart par puit pour le clonage en dilutions limites des virus FCV ou de 1 ml par Falcon.
Lorsque l'effet cytopathique (ECP) est complet (24-48 heures après le début de la mise en culture), les suspensions virales sont récoltées et congelées à -70°C. 3 à 4 passages successifs sont généralement nécessaires à la production d'un lot viral. Le lot viral est stocké à -70°C.

### Exemple 3 : Production du sérum

Pour chaque virus FCV, un antisérum est produit par inoculation de chatons par la voie oronasale avec 10^{6.0} DICC₅₀ du virus FCV concerné. Les chatons exempts de pathogènes spécifiques (EOPS) sont âgés de 10 à 14 semaines.
Le sérum obtenu pour chaque animal est collecté un mois après l'infection. Les sérums sont inactivés par la chaleur (30 minutes à 56°C), répartis, aliquotés et stockés à -20°C.

### Exemple 4 : Séroneutralisation croisée in vitro

Des tests de séroneutralisation croisée ont été effectués entre 18 isolats de terrain obtenus par écouvillonnages pharyngés pratiqués sur des chats présentant des signes de calicivirose féline. 7 ont pour origine géographique la France, il s'agit des isolats identifiés A2, F3031, G1, G3, F1, H3-2 et H1-4, 4 ont pour origine géographique la Grande-Bretagne, il s'agit des isolats identifiés J5, 337, 388b et 431. Enfin, 7 ont pour origine géographique les USA, il s'agit des isolats identifiés RMI1, RMI2, RMI3, RMI5, RMI6, RMI7 et RMI9.
Le sérum obtenu pour chaque isolat (exemple 3) est testé pour son aptitude à neutraliser les 18 isolats. Les sérums sont dilués en cascade au tiers avec du milieu DMEM dans des plaques à 96 puits pour culture cellulaire. A 0,05 ml du sérum dilué sont ajoutés 0,05 ml de milieu de culture contenant approximativement 100 DICC₅₀ de la souche virale sélectionnée produite comme à l'exemple 2. Ce mélange est incubé pendant 2 heures à 37°C en étuve en atmosphère contenant 5% CO₂.
0,15 ml d'une suspension de cellules CRFK contenant environ 100 000 cellules par ml est ensuite ajoutée à chaque mélange. L'effet cytopathique est observé par microscopie à contraste de phase après 4 jours de culture à 37°C en atmosphère contenant 5% CO₂. Les titres neutralisants de chaque sérum sont calculés d'après la méthode de Kärber. Les titres sont donnés sous la forme de la dilution la plus importante inhibant l'effet cytopathique pour 50 % des cupules. Les titres sont exprimés en log₁₀. Le titre minimum ainsi trouvé est de 0,7 log₁₀ VN₅₀. Chaque sérum est titré au moins deux fois, de préférence trois fois.

### Exemple 5 : Tests d'immunofluorescence indirecte (IFI)

Les tests IFI sont effectués sur des plaques 96 puits contenant les cellules CRFK cultivées en monocouches et infectées par les virus FCV à tester.
200 µl par cupule d'une suspension de cellules CRFK à 90 000 cellules/ml en milieu F15 (Gibco BRL, Cat # 045-1075) contenant 5 % de sérum de veau foetal sont mises en culture en plaque de 96 cupules. A la confluence, 320 DICC50 de FCV sont inoculés sous 100 µl de milieu F15. Lorsque les premiers foyers d'ECP apparaissent, les cellules sont alors rincées par du PBS sans calcium ni magnésium (PBS⁻, Sigma) froid, puis fixées à -20°C pendant 30 minutes par de l'acétone froid contenant 5 % v/v d'eau. Après séchage, les cellules infectées et fixées sont mises en contact pendant 30 minutes à 37°C avec 100 µl par cupule de liquide d'ascite correspondant à l'anticorps monoclonal 44 anti-FCV 431 (hybridome 431 2 0 17 E9 T), dilué au 1/5000 ème environ dans du tampon TRIS-HCl 50 mM pH7,6.
Après deux rinçages en PBS⁻, la fixation des anticorps est révélée par incubation dans les mêmes conditions d'un anticorps de chèvre anti-IgG de souris conjugué à de l'isothianate de fluorescéine (Biosys, conjugué FITC à 2mg/ml) et dilué à 1/150 en tampon TRIS-HCl 50 mM pH7,6. La lecture est faite sous microscope optique en lumière UV.
Cet anticorps monoclonal a été testé vis-à-vis de chacun des isolats du panel. Il s'est fixé exclusivement sur les cellules CRFK infectées par le FCV 431.
Ce test peut être utilisé pour déterminer les équivalents de la souche FCV 431. Ces équivalents sont ceux sur lesquels se fixe l'anticorps monoclonal 44.

### Exemple 6 : Synergie

32 chatons EOPS non vaccinés de 9 semaines environ sont répartis par randomisation en 4 groupes (identifiés de A à D) de 8 chatons chacun, chaque groupe est hébergé dans un box isolé.
Après décongélation des suspensions virales (exemple 2) et dilution en PBS de manière à obtenir le titre souhaité, les chats sont vaccinés par injection sous-cutanée de 1 ml d'inoculum FCV G1 à 10^{3,3} DICC₅₀/ml pour le groupe B, de 1 ml d'inoculum FCV 431 à 10^{3,5} DICC₅₀/ml pour le groupe C, de 0,5 ml de l'inoculum FCV G1 à 10^{3,3} DICC₅₀/ml et 0,5 ml de l'inoculum FCV 431 à 10^{3,5} DICC₅₀/ml (en un point d'injection différent) pour le groupe D. Le groupe A sert de groupe témoin.
La moitié de chaque groupe A à D est répartie au hasard en deux groupes 1 et 2 et hébergée dans des boxes séparés. Les animaux sont éprouvés au 31^{ème} jour après vaccination ( J31).
Les animaux de groupe 1 sont éprouvés par administration de 1 ml de souche virale d'épreuve FCV 220 titrant 10^{7,2} DICC₅₀/ml par voie oronasale (0,5 ml par voie orale et 0,25 ml dans chaque narine).
Les animaux de groupe 2 sont éprouvés par administration de 1 ml de souche virale d'épreuve FCV 393 titrant 10^{6,8} DICC₅₀/ml par voie oronasale (0,5 ml par voie orale et 0,25 ml dans chaque narine).
Les souches virulentes FCV 220 et 393 ont été choisies car elles sont éloignées en séroneutralisation croisée des souches virales FCV G1 et 431.
Toute contamination croisée entre les deux boxes est soigneusement évitée. Le suivi clinique des animaux de deux groupes se fait par prises de la température rectale et examens cliniques des animaux (état général, présence d'ulcères de la langue et du palais, présence de gingivite, présence de rhinite, présence de conjonctivite, présence de boiterie, décès de l'animal).
Le score clinique total de chaque animal fut calculé en additionnant les scores obtenus pour chaque groupe de signes cliniques selon le barème suivant :
- température rectale :
   0 - inférieure à 39°C
   1 - supérieure ou égale à 39°C et inférieure à 39,5°C
   2 - supérieure ou égale à 39,5°C et inférieure à 40°C
   3 - supérieure ou égale à 40°C
- état général :
   0 - comportement normal
   1 - abattement
- ulcères de la langue et du palais (somme des diamètres de tous les ulcères, s'il y en a plusieurs) :
   0 - absence d'ulcère
   1 - diamètre de 1 à 5 mm
   2 - diamètre de 6 à 10 mm
   3 - diamètre supérieur à 10 mm
- gingivite :
   0 - absence de gingivite
   1 - gingivite
- rhinite :
   0 - absence de rhinite
   1 - rhinite à écoulement nasal séreux
   2 - rhinite à écoulement nasal muqueux à mucopurulent
- conjonctivite :
   0 - absence de conjonctivite
   1 - conjonctivite à écoulement séreux
   2 - conjonctivite à écoulement mucopurulent
- boiterie :
   0 - absence de boiterie
   1 - boiterie
- décès :
   0 - survie
   5 - décès.

Les scores cliniques moyens obtenus sont les suivants :

| Groupe/épreuve | FCV 220 | FCV 393 |
|---|---|---|
| Témoins (groupe A) | 31 | 30 |
| FCV G1 (groupe B) | 5 | 23 |
| FCV 431 (groupe C) | 6 | 18 |
| FCV G1 + FCV 431 (groupe D) | 2 | 9 |

Les résultats ainsi obtenus montrent une synergie entre les souches FCV G1 et FCV 431 par une différence significative entre la valeur moyenne obtenue pour la meilleure des souches et celle obtenue pour l'association des deux souches (test de Kruskal-Wallis).

### Exemple 7 : Production du vaccin inactivé

Les cellules CRFK sont cultivées à 37°C en flacons roulants de 2 litres (850 cm²) en milieu Eagle modifié (MEM, Gibco BRL) supplémenté avec 2,5 % d'hydrolysat de lactalbumine (Gibco BRL) et de 5% de sérum de veau foetal (Gibco BRL). On ajoute par flacon roulant 300 ml d'une suspension cellulaire en milieu MEM à environ 100 000 cellules/ml. Après 3 jours la couche cellulaire est confluente. Le milieu de culture cellulaire est alors remplacé par du milieu MEM sans sérum et le virus FCV ajouté à une multiplicité d'infection (moi) de 0,5 DICC₅₀/cellule. La culture virale est maintenue à 37°C pendant 24 à 48 heures jusqu'à l'obtention d'un effet cytopathique pour l'ensemble du tapis cellulaire. La suspension virale est récoltée puis clarifiée sur un filtre poche de porosité 1,5 µm. Le titre du virus FCV à la récolte est 8,5 +/- 0,3 log10 DICC₅₀/ml.
Le virus est inactivé par l'éthylèneimine à la concentration d'environ 8 mM à 22°C pendant 18 heures.
L'éthylèneimine est préparé extemporanément en dissolvant 28 g de soude en pastille dans 200 ml d'eau distillée et en ajoutant 68,1 g de bromoéthylamine (BEA) correspondant à une solution 1,2 M environ (H. Bahnemann, Arch. Virol., 1975, **47**, 47-56). La suspension virale inactivée est concentrée 100 fois sur cassette d'ultrafiltration type Ultrasette avec un seuil de coupure de 100 kDa (Filtron) puis congelée à -70°C.

La suspension virale inactivée après décongélation est diluée au 1/33^{ème} en tampon PBS (NaCl 8g/l ; KCl 0,2 g/l ; KH₂PO₄ 0,2 g/l ; Na₂HPO₄, 2 H₂O 1,44 g/l). Le vaccin est préparé de la manière suivante : 167 ml de phase aqueuse constituée de la dilution du virus inactivé sont émulsionnés dans 83 ml de phase huileuse contenant 7% p/v d'oléate d'anhydromannitol, 8% p/v d'acide oléique éthoxylé à 11 molécules d'oxyde d'éthylène (OE) en moyenne et 85% v/v d'huile de paraffine liquide légère (type Pharmacopée européenne) à l'aide d'un émulseur à turbine Silverson à 32°C pendant 2 minutes. Le vaccin est ensuite stocké à 5°C.

Une méthode alternative pour préparer le vaccin consiste à mettre en émulsion par trois passages à travers un homogénéiseur haute pression modèle Y110 (Microfluidics Corp.) à une pression de 600 bars et une température comprise entre 30 et 40°C le mélange 5% p/v squalane, 2,5% p/v Pluronic® L121, 0,2% p/v Tween 80, 92,3% v/v de suspension virale inactivée diluée au 1/46^{ème} en tampon PBS après décongélation. Le vaccin est ensuite stocké à 5°C.

Une autre méthode alternative consiste à réaliser une solution à 0,4% p/v de Carbopol® 974P en eau physiologique (NaCl 9 g/l). Le pH est ajusté à 7,3-7,4 avec de la soude. Cette solution de Carbopol est ensuite mélangée à partie égale à la suspension de virus FCV inactivé diluée au 1/25^{ème} après décongélation. Le vaccin est ensuite conservé à 5°C.

La phase aqueuse des émulsions ou la phase aqueuse mélangée au Carbopol® est constituée d'une dilution en PBS de la suspension virale inactivée concentrée correspondant soit à la souche FCV 431 soit à la souche FCV G1 soit un mélange à partie égale des souches FCV 431 et G1.

### Exemple 8 : Contrôle de l'immunogénicité du FCV 431 inactivé

19 chatons EOPS non vaccinés de 9 semaines environ sont répartis par randomisation en 2 groupes (identifiés de A et B), le premier de 12 chatons et le second de 7 chatons, chaque groupe est hébergé dans un box isolé.
Le vaccin est préparé avec l'adjuvant composé d'oléate d'anhydromannitol, d'acide oléique éthoxylé et d'huile de paraffine liquide légère tel que décrit à l'exemple 7.
Les chats sont vaccinés à deux reprises (J0 et J28) par injection sous-cutanée de 1 ml d'inoculum FCV 431 à 10⁷ DICC₅₀/ml pour le groupe A. Le groupe B sert de groupe témoin.
Les animaux sont éprouvés au 42^{ème} jour après primovaccination (J42) par administration de 1 ml de souche virale d'épreuve FCV 431 titrant 10⁶ DICC₅₀/ml par voie oronasale (0,5 ml par voie orale et 0,25 ml dans chaque narine).

Le taux en anticorps neutralisants anti-FCV 431 et le score clinique ont eté suivis. Le score clinique total de chaque animal a été calculé en additionnant les scores obtenus pour chaque groupe de signes cliniques selon le barème donné à l'exemple 6.
Les résultats obtenus sont les suivants :

Titres en anticorps neutralisants anti-FCV 431 exprimés en log₁₀ VN₅₀/ml :

| Groupe | Anticorps à J0 | Anticorps à J28 | Anticorps à J42 |
|---|---|---|---|
| vaccin FCV431 (groupe A) | 0,24 | 1,61 - | 2,87 |
| témoins (groupe B) | 0,24 | 0,24 | 0,24 |

Scores cliniques moyens sur la période J42 à J56 :

| Groupe | Score clinique |
|---|---|
| vaccin FCV 431 (groupe A) | 0,7 |
| témoins (groupe B) | 33,7 |

Ces résultats montrent une excellente protection clinique contre l'épreuve homologue et une bonne séroconversion.

## Revendications

1. Préparation immunogène ou vaccin contre la calicivirose féline, comprenant un calcivirus félin (FCV) ayant la particularité d'être reconnu par l'anticorps monoclonal 44 sécrété par l'hybridome déposé à la CNCM sous le numéro d'accès I-2282, ou la protéine de capside de ce FCV, et un véhicule ou excipient acceptable sur le plan vétérinaire.

2. Préparation immunogène ou vaccin selon la revendication 1, comprenant un calicivirus félin (FCV) isolé ayant la particularité d'être reconnu par l'anticorps monoclonal 44 sécrété par l'hybridome déposé à la CNCM sous le numéro d'accès I-2282.

3. Préparation immunogène ou vaccin selon la revendication 1 ou 2, dans lequel le FCV est inactivé.

4. Préparation immunogène ou vaccin selon l'une quelconque des revendications 1 à 3, comprenant un FCV de la souche 431 telle que déposée à la CNCM sous le numéro d'accès CNCM I-2166.

5. Préparation immunogène ou vaccin selon l'une quelconque des revendications 1 à 4, qui comprend la protéine de capside d'un FCV ayant la particularité d'être reconnu par l'anticorps monoclonal 44 sécrété par l'hybridome déposé à la CNCM sous le numéro d'accès I-2282.

6. Préparation immunogène ou vaccin selon la revendication 5, dans lequel la protéine de capside provient d'un FCV de la souche 431 telle que déposée à la CNCM sous le numéro d'accès CNCM I-2166.

7. Préparation immunogène ou vaccin selon l'une quelconque des revendications 1 à 6, comprenant en outre un antigène provenant d'une autre souche de calicivirus félin (FCV).

8. Préparation immunogène ou vaccin selon la revendication 7, dans lequel l'autre souche FCV est la souche G1 telle que déposée à la CNCM sous le numéro d'accès CNCM I-2167.

9. Préparation immunogène ou vaccin selon la revendication 7 ou 8, comprenant cet autre calicivirus félin (FCV) sous forme inactivée.

10. Préparation immunogène ou vaccin selon l'une quelconque des revendications 7 à 9, comprenant des sous-unités de cet autre FCV.

11. Préparation immunogène ou vaccin selon l'une quelconque des revendications 7 à 10, comprenant de la protéine de capside de cet autre FCV.

12. Préparation immunogène ou vaccin selon l'une quelconque des revendications 1 à 11, comprenant en outre au moins une autre valence vaccinale féline induisant une réponse immunitaire contre au moins un autre pathogène félin.

13. Préparation immunogène ou vaccin selon la revendication 12, dans lequel le ou les autres pathogènes félins est/sont choisi(s) parmi les virus herpès félins (FHV), le virus de la leucémie féline (FeLV), le virus de la panleucopénie féline (FPV), le virus de la péritonite infectieuse féline (FIPV), le virus de l'immunodéficience féline (FIV), le virus de la rage et *Chlamydia*.

14. Préparation immunogène ou vaccin selon la revendication 12 ou 13, comprenant une autre valence vaccinale féline sous forme de vaccin vivant atténué, vaccin inactivé, vaccin sous-unitaire, vaccin recombiné ou vaccin polynuctéotidique.

15. Préparation immunogène ou vaccin selon l'une quelconque des revendications 1 à 14, comprenant en outre un adjuvant.

16. Préparation immunogène ou vaccin selon la revendication 15, comprenant de l'hydroxyde d'alumine.

17. Préparation immunogène ou vaccin selon la revendication 15, comprenant un polymère de l'acide acrylique ou méthacrylique ou un polymère d'anhydride maléique et de dérivé alcényle.

18. Préparation immunogène ou vaccin selon la revendication 17, comprenant un carbomère.

19. Préparation immunogène ou vaccin selon l'une quelconque des revendications 1 à 14, sous forme d'une émulsion huile-dans-l'eau.

20. Hybridome tel que déposé à la CNCM sous le numéro d'accès CNCM. I-2282.

21. Anticorps monoclonal susceptible d'être produit à partir de l'hybridome selon la revendication 20.

## Patentansprüche

1. Immunogene Zubereitung oder Impfstoff gegen die Katzen-Calicivirus-Erkrankung, umfassend ein felines Calicivirus (FCV) mit der Besonderheit, dass es vom monoclonalen Antikörper 44 erkannt wird, der von dem Hybridom sezerniert wird, das unter der Hinterlegungsnummer I-2282 bei der CNCM (Collection Nationale de Cultures de Microorganismes) hinterlegt wurde, oder das Capsidprotein des FCV und einen Träger oder Exzipienten, der veterinärmedizinisch verträglich ist.

2. Immunogene Zubereitung oder Impfstoff nach Anspruch 1, umfassend ein isoliertes felines Calicivirus (FCV) mit der Besonderheit, dass es vom monoclonalen Antikörper 44 erkannt wird, der von dem Hybridom sezerniert wird, das unter der Hinterlegungsnummer I-2282 bei der CNCM hinterlegt wurde.

3. Immunogene Zubereitung oder Impfstoff nach Anspruch 1 oder 2, bei dem das FCV inaktiviert ist.

4. Immunogene Zubereitung oder Impfstoff nach einem der Ansprüche 1 bis 3, umfassend das FCV des Stammes 431, so wie er bei der CNCM unter der Hinterlegungsnummer CNCM I-2166 hinterlegt wurde.

5. Immunogene Zubereitung oder Impfstoff nach einem der Ansprüche 1 bis 4, umfassend das Capsidprotein eines FCV mit der Besonderheit, dass es vom monoclonalen Antikörper 44 erkannt wird, der von dem Hybridom sezerniert wird, das unter der Hinterlegungsnummer I-2282 bei der CNCM hinterlegt wurde.

6. Immunogene Zubereitung oder Impfstoff nach Anspruch 5, wobei das Capsidprotein von einem FCV des Stammes 431 abstammt, so wie er bei der CNCM unter der Hinterlegungsnummer CNCM I-2166 hinterlegt wurde.

7. Immunogene Zubereitung oder Impfstoff nach einem der Ansprüche 1 bis 6, zusätzlich umfassend ein Antigen, das von einem anderen Stamm des felinen Calicivirus (FCV) abstammt.

8. Immunogene Zubereitung oder Impfstoff nach Anspruch 7, wobei der andere FCV-Stamm der Stamm G1 ist, so wie er bei der CNCM unter der Hinterlegungsnummer CNCM I-2167 hinterlegt wurde.

9. Immunogene Zubereitung oder Impfstoff nach einem der Ansprüche 7 oder 8, umfassend das andere feline Calicivirus (FCV) in inaktivierter Form.

10. Immunogene Zubereitung oder Impfstoff nach einem der Ansprüche 7 bis 9, umfassend Untereinheiten des anderen FCV.

11. Immunogene Zubereitung oder Impfstoff nach einem der Ansprüche 7 bis 10, umfassend das Capsidprotein des anderen FCV.

12. Immunogene Zubereitung oder Impfstoff nach einem der Ansprüche 1 bis 11, weiterhin umfassend mindestens eine andere feline Impfstoffvalenz, die eine Immunantwort gegen mindestens einen anderen felinen Krankheitserreger hervorruft.

13. Immunogene Zubereitung oder Impfstoff nach Anspruch 12, wobei der oder die anderen felinen Krankheitserreger ausgewählt ist/sind aus felinem Herpesvirus (FHV), felinem Leukämievirus (FeLV), felinem Panleukopenie-Virus (FPV), felinem infektiösem Peritonitis-Virus (FIPV), felinem Immundefizienzvirus (FIV), Tollwutvirus und *Chlamydia.*

14. Immunogene Zubereitung oder Impfstoff nach Anspruch 12 oder 13, umfassend eine andere feline Impfstoffvalenz in Form eines abgeschwächten Lebendimpfstoffs, eines inaktivierten Impfstoffs, einer Impfstoffuntereinheit, eines rekombinanten Impfstoffs oder eines Polynucleotidimpfstoffs.

15. Immunogene Zubereitung oder Impfstoff nach einem der Ansprüche 1 bis 14, weiterhin umfassend einen Hilfsstoff.

16. Immunogene Zubereitung oder Impfstoff nach Anspruch 15, umfassend Aluminiumhydroxid.

17. Immunogene Zubereitung oder Impfstoff nach Anspruch 15, umfassend ein Acrylsäure- oder Methacrylsäurepolymer oder ein Polymer aus Maleinanhydrid und einem Alkenylderivat.

18. Immunogene Zubereitung oder Impfstoff nach Anspruch 17, umfassend ein Carbomer.

19. Immunogene Zubereitung oder Impfstoff nach einem der Ansprüche 1 bis 14 in Form einer Öl-in-Wasser-Emulsion.

20. Hybridom wie bei der CNCM unter der Hinterlegungsnummer CNCM I-2282 hinterlegt.

21. Monoclonaler Antikörper, der von dem Hybridom nach Anspruch 20 produziert werden kann.

## Claims

1. Immunogenic preparation or vaccine against the feline calicivirus comprising a feline calicivirus (FCV) having the particularity of being recognized by the monoclonal antibody 44 secreted by the hybridoma deposited at the CNCM under the access number 1-2282, or the capsid protein of this FCV, and a vehicle or excipient which is acceptable in veterinarian medicine.

2. Immunogenic preparation or vaccine according to claim 1, comprising an isolated feline calicivirus (FCV) having the particularity of being recognized by the monoclonal antibody 44 secreted by the hybridoma deposited at the CNGM under the access number 1-2282.

3. Immunogenic preparation or vaccine according to claim 1 or 2, in which the FCV is inactivated.

4. Immunogenic preparation or vaccine according to any one of claims 1 to 3, comprising an FCV of the 431 strain as deposited at the ONOM under the access number ONOM 1-2166.

5. Immunogenic preparation or vaccine according to any one of claims 1 to 4, which comprises the capsid protein of an FCV having the particularity of being recognized by the monoclonal antibody 44 secreted by the hybridoma deposited at the CNCM under the access number 1-2282.

6. immunogenic preparation or vaccine according to claim 5 in which the capsid protein originates from an FCV of the 431 strain as deposited at the ONOM under the access number ONOM 1-2166.

7. Immunogenic preparation or vaccine according to any one of claims 1 to 6, also comprising an antigen originating from another strain of feline calicivirus (FCV).

8. Immunogenic preparation or vaccine according to claim 7, in which the other FCV strain is the Gi strain as deposited at the ONOM under the access number ONOM 1-2167.

9. Immunogenic preparation or vaccine according to claim 7 or 8, comprising said other feline calicivirus (FCV) in inactivated form.

10. Immunogenic preparation or vaccine according to any one of claims 7 to 9, comprising subunits of said other FCV.

11. Immunogenic preparation or vaccine according to any one of claims 7 to 10, comprising the capsid protein of said other FCV.

12. Immunogenic preparation or vaccine according to any one of claims 1 to 11, also comprising at least one other feline vaccine valence inducing an immune response to at least one other feline pathogen.

13. Immunogenic preparation or vaccine according to claim 12 in which the other feline pathogen or pathogens is/are chosen from the feline herpes virus (FHV), the feline leukaemia virus (FeLV), the feline panleucopenia virus (FPV), the feline infectious peritonitis virus (FIPV), the feline immunodeficiency virus (Fly), the rabies virus and *Chiamycila.*

14. Immunogenic preparation or vaccine according to claim 12 or 13, comprising another feline vaccine valence in the form of a live attenuated vaccine, an inactivated vaccine, a subunit vaccine, a recombinant vaccine or a polynucleotide vaccine.

15. Immunogenic preparation or vaccine according to any one of claims I to 14 also comprising an adjuvant,

16. Immunogenic preparation or vaccine according to claim 15, comprising aluminium hydroxide.

17. Immunogenic preparation or vaccine according to claim 15, comprising an acrylic or methacrylic acid polymer or a polymer of maleic anhydride and an alkenyl derivative.

18. Immunogenic preparation or vaccine according to claim 17, comprising a carbomer.

19. Immunogenic preparation or vaccine according to any one of claims 1 to 14 in the form of an oil-in-water emulsion.

20. Hybridoma as deposited at the CNCM under the access number CNCM 1-2282.

21. Monoclonal antibody which can be produced from the hybridoma according to claim 20.
